# EUROPEAN PATENT SPECIFICATION

(11) **EP 3 139 995 B1**
(45) Date of publication and mention of the grant of the patent: **21.03.2018**
(21) Application number: 15726722.0
(22) Date of filing: 04.05.2015
(51) Int. Cl.: A61N 1/05, A61N 1/32, A61B 18/00, A61N 1/04, A47K 11/00, A47K 11/10

(54) **REMOTELY CONTROLLED PORTABLE DEVICE FOR WRINKLES' TREATMENT BY MEANS OF AMPLITUDE MODULATED CURRENT**
FERNGESTEUERTE TRAGBARE VORRICHTUNG ZUR FALTENBEHANDLUNG MITTELS AMPLITUDENMODULIERTEM STROM
DISPOSITIF PORTATIF TÉLÉCOMMANDÉ POUR LE TRAITEMENT DES RIDES AU MOYEN D'UN COURANT MODULÉ EN AMPLITUDE

(30) Priority: 07.05.2014 IT NA20140013
(43) Date of publication of application: 15.03.2017
(73) Proprietor: Brera Medical Technologies S.r.l., 84061 Ogliastro Cilento (SA) (IT)
(72) Inventor: GRATTACASO, Attilio, 84043 Agropoli (SA) (IT)
(74) Representative: Conversano, Gabriele
(86) International application number: PCT/IB2015/053247
(87) International publication number: WO 2015/170241

(56) References cited:
- WO-A1-2014/004644
- US-A1- 2006 089 688
- US-A1- 2007 032 840
- US-A1- 2014 081 118
- US-B1- 6 181 974

## Description

Object of the present invention is a device for treating wrinkles by means of unidirectional currents.

### BACKGROUND OF THE INVENTION

The present invention relates to the technical field which comprises devices used for aesthetic treatments of the body. In particular, it relates to electro-medical devices for treating wrinkles.

It is well known that our society is based on the image. Photography, cinema and above all television have contributed to give particular importance to the exterior appearance and to how one presents oneself: the look, the hairstyle, the make up, the youthful appearance. Moreover, considering the exponential increase of cosmetics for men, giving attention to ones body is no more peculiar only to women. The use of products and solutions allowing us to improve our aesthetical look is gratifying, we feel better and safer in the interpersonal relations as well as in the relation with ourselves.

Therefore, at the state of the art there are known and are commonly used electro-medical devices which comprise electrodes configured to be surface applied on the skin in the area of the wrinkle to be treated, and to send low frequency, amplitude modulated, mono-directional currents. These currents generate an inflammatory reaction on the interested area causing wrinkle leveling. The treatment has an immediate (generally, the wrinkle disappears nearly in about two minutes) but temporary effect: after about seventy-two hours the inflammatory reaction disappears and the wrinkle comes out again. Anyway, even if the single application has a temporary effect, a time prolonged treatment has therapeutic effects which tend to make the wrinkle disappear.

### STATE OF THE ART

Currently, the aesthetic treatments described are possible only in aesthetic centers having electro-medical devices intended for this aim. Such devices are usually very cumbersome and for their usage it is needed the assistance of a specialized technician, who is expert in using such apparatuses, since it is quite difficult to control the parameters regulating the currents to be sent to the electrodes, which are function of the kind of wrinkle and skin.

For this reason, the electro-medical treatments of imperfections caused by wrinkles are characterized by high costs and not always satisfying results.

There exist devices with similar aim but of portable kind as well. Obviously, for regulating the parameters of the currents to be sent to the electrodes on the basis of the kind of wrinkle and skin, it is essential that they are provided with a little display, of such dimensions that they are portable; moreover, the information which can be provided to the user is limited; in fact, it is not possible to introduce a smart unit therein since the device would increase dimensions and costs inevitably.

Even if the application is simple in itself since it is needed only to arrange the electrodes on the area to be treated and to exert a light pressure so that these ones penetrate the skin in surface, the usage of the devices is not yet for all: it is needed, in fact, that the not expert user becomes friendly with the regulating commands of the electro-medical device.

Yet, there exist portable and simply usable devices which however do not offer the possibility to manage the regulation parameters of the current to be sent to the electrodes; this solution, even if valid for usage easiness, does not guarantee an optimal treatment of the wrinkle since the treatment parameters remain the same during all its duration.

An example of such portable devices is described in the document US2007/0032840, but other similar devices are known with substantially the same just described limits.

### OBJECT OF THE INVENTION

Therefore, aim of the present invention is to provide an electro-medical device for treating wrinkles by means of application of amplitude modulated, mono-directional currents, which overcomes the limits linked to the devices known at the state of the art and, in particular, which is more user-friendly with respect to the portable devices known at the state of the art, even if they allow to personalize efficiently the treatment in function of the kind of wrinkle to be treated. According to another aim the object of the present invention is intended to provide a device which is less cumbersome and cheaper than the devices currently used in the aesthetic centers, even if it maintains the same efficacy level and, at the same time, makes it simpler for a not professional user to use the device.

According to another aim the object of the present invention is intended to provide an electro-medical device whose electrodes are configured so that they cannot cause damages to the skin of the subject treated, also when the device is used by a not expert person. Yet, according to another aim the present invention is intended to provide an electro-medical device for the treatment of wrinkles which is simpler to be handled, less cumbersome and more comfortable to be used. The invention is defined in claim 1. Further aspects and preferred embodiments are defined in the dependent claims.

### SUMMARY

The present invention reaches the prefixed aims since it is a portable device for treating wrinkles by means of amplitude modulated, mono-directional currents, comprising: an outer case of such dimensions that it can be grasped only by a hand, a plurality of electrodes of little dimensions which come out from one of the surfaces of said case, each one of said electrodes being configured to slide along its own axis inside a respective guide and to be pushed outwards by the action of a spring; a programmable electronic unit, arranged inside said case, configured to generate low frequency, amplitude modulated, mono-directional currents, and to send them to said electrodes; a battery, arranged inside said case, configured to feed said programmable electronic unit, characterized in that each one of said electrodes, in its own terminal portion, is provided with a circular crown of pointed terminals and with another needled point arranged substantially at the axis of the electrode and projecting with respect to the plane of said pointed circular crown and in that said device further comprises a wireless data transmission unit, arranged inside the box, configured to manage the regulation parameters of said currents sent to the electrodes.

### DETAILED DESCRIPTION OF THE INVENTION

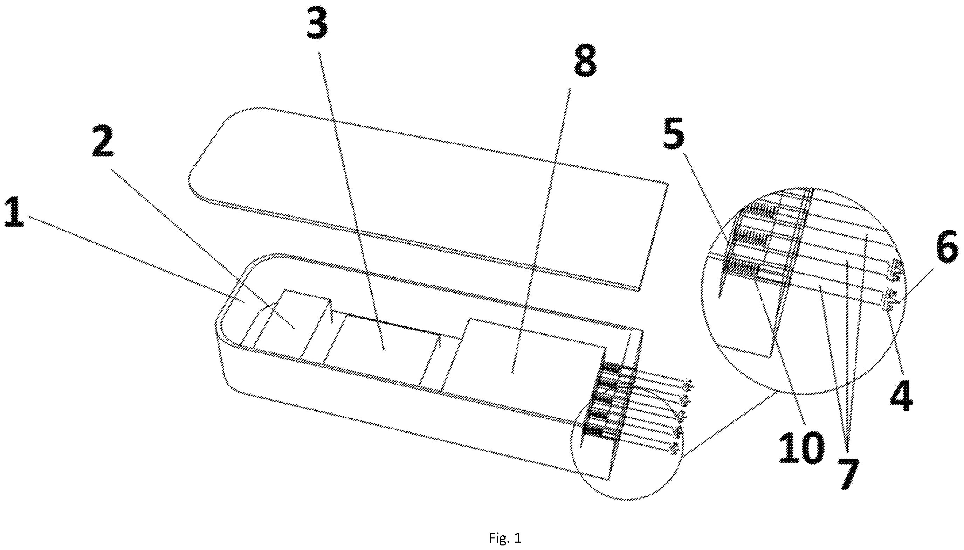

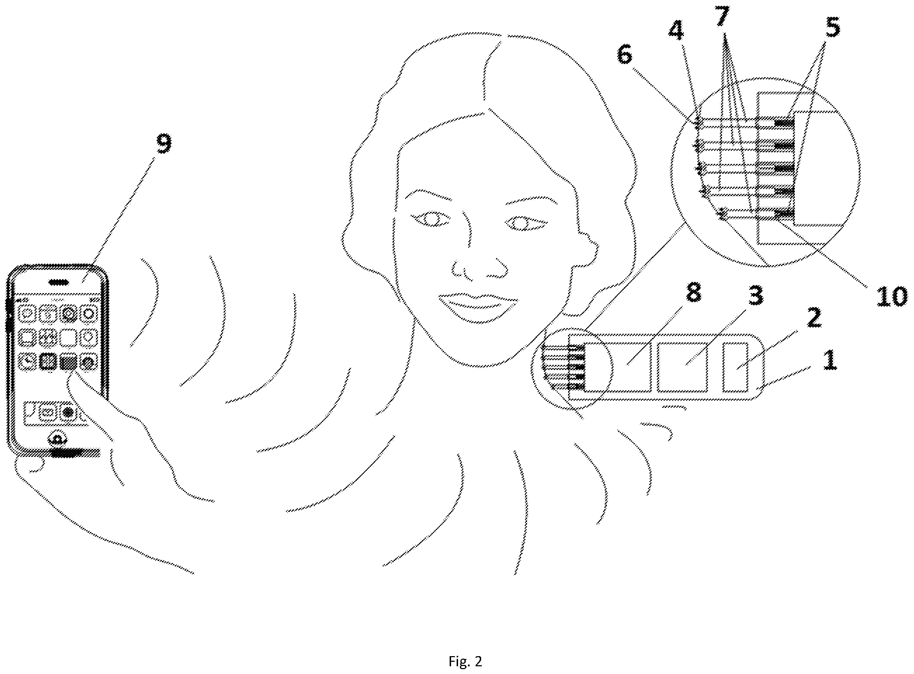

The object of the present invention will be described in the following by means of a preferred embodiment thereof, with reference to the appended figures 1 and 2.
Figure 1 shows an exploded view of the device and respective configuration of the electrodes;
Figure 2 shows a preferred mode of application of the device for treating wrinkles.

As it is shown in figure 2, the object of the present invention comprises a device for treating wrinkles by means of amplitude modulated, mono-directional currents, which is controllable by means of a smartphone, tablet or similar device (9).

Recently, the cellular technology has diffused so dizzily that the so-called cellular telephones of smartphone kind are available for all kinds of users. The smartphone is a cellular telephone based on an operating system for mobile devices, with much more advanced computing and connection capacities over the normal cellular telephones. For the smartphones there always exist newer accessories functioning thanks to the so-called applications, i.e. simplified software with a group of computing instructions, designed to make possible a service or a series of services or tools considered useful or desirable by the user.

The smartphones represent by now a technology available for every one, and the respective apps, i.e. variants of the computing applications, dedicated to the devices of mobile kind, such for example smartphones and tablets, have become user-friendly, interactive and universally accessible.

With reference to the device, the same comprises an outer case (1), preferably but not limitingly shaped as a parallelepiped, of such dimensions that it is grasped only by a hand, and provided with a plurality of electrodes (7) which come out from one of the surfaces of said outer case. The electrodes are in metal material, while the outer case is preferably realized in plastic material.

Said electrodes (7) are of little dimensions (few centimeters in length) and, in their terminal portion, they are provided with a circular crown (4) of pointed terminals, and with another needled point (6) arranged substantially at the axis of the electrodes and projecting with respect to the plane of said pointed circular crown. The circular crown (4), since it is arranged on a plane lightly withdrawn with respect to the needled point (6), does not allow the same to penetrate the skin more than needed, thus avoiding useless bleeding of the treated zone. This fact allows a not expert to use the device.

The electrodes (7) slide along their own axis inside guides (10). A spring (5) is arranged inside the box at each electrode and is configured so that the respective electrode is pushed outwards. In this way, as it is shown in figure 2, the electrodes (7) can be adapted to the irregular surface on which they are applied (the neck in the case shown in figure, but similarly on other parts of the body), retracting inside the guides (10) but maintaining the contact on the skin constantly, thanks to the springs (5) pushing outwards.

The device further comprises:
- a programmable electronic unit (8), arranged inside said case (1), configured to generate low frequency, amplitude modulated, mono-directional currents and to send them to the electrodes (7),
- a wireless data transmission unit (3), arranged inside said case (1) as well, which manages the regulation parameters of the currents which said electronic unit (8) sends to the electrodes (7). Said data transmission unit (3) is configured so that it receives the configuration parameters of the currents from a remote controller, preferably a cellular telephone of smartphone kind (9) or similar device;
- a battery (2), preferably of rechargeable kind, arranged inside said case (1) as well, and configured so that the programmable electronic unit (8) and the wireless data transmission unit (3) are fed.

Obviously the fact that the portable device for treating wrinkles is controlled by a remote controller (the smartphone) simplifies its structure, thus making the device free of both the display and the smart unit for control and regulation. These modifications allow to obtain a cheaper, of less dimensions, less weight and definitely more comfortable device to be used.

Thanks to a simple software application, specifically created with the aim of making the portable device functional for treating wrinkles, such tool becomes user-friendly. In fact, by means of the smartphone, the user can see the instructions in video format in real time. Moreover, the regulation parameters of the currents to be sent to the electrodes are simply controlled, interactive and intuitive.

The usage of a smartphone or similar device avoids also the limits in designing the interface linked to the usage of smart units integrated inside the device which, for clear reasons of inexpensiveness and display dimensions, allow a very limited interaction between user and device.

By using the portable device for treating wrinkles by means of smartphones, the user becomes immediately friendly with the device for treating wrinkles, avoiding each difficulty of use of the same. This is what makes this technology accessible to every one.

This innovative device offers the possibility to carry out a valid treatment which allows to reach the leveling effect of the wrinkles immediately and totally independently. The easiness of use of this little device is equal to the one of a cosmetic of common use: it is sufficient to acquire a minimum manual skill to be able to use it on the face with the same easiness with which we apply a corrector, a pencil or a lipstick. The user, thanks to clear instructions provided instantly by the software interface, can use the device in correct way and without any risks.

Moreover, the user having this device, by paying attention to the respective use destination, usage modes, instructions, indications and safety rules, can undergo a treatment every time it is desired and without further costs. This implies the possibility to carry out treatments in total freedom and definitely affording contained costs.

## Claims

1. Portable device for treating wrinkles by means of amplitude modulated, mono-directional currents, comprising:
- an outer case (1) of such dimensions that it can be grasped only by a hand,
- a plurality of electrodes (7) of little dimensions which come out from one of the surfaces of said case (1), each one of said electrodes being configured to slide along its own axis inside a respective guide (10) and to be pushed outwards by the action of a spring (5);
- a programmable electronic unit (8), arranged inside said case (1), configured to generate low frequency, amplitude modulated, mono-directional currents, and to send them to said electrodes (7);
- a battery (2), arranged inside said case (1), configured to feed said programmable electronic unit (8),
**characterized in that**
each one of said electrodes (7), in its own terminal portion, is provided with a circular crown (4) of pointed terminals and with another needled point (6) arranged substantially at the axis of the electrode (7) and projecting with respect to the plane of said pointed circular crown (4),
and **in that**
said device further comprises a wireless data transmission unit (3), arranged inside the box (1), configured to manage the regulation parameters of said currents sent to the electrodes (7).

2. Portable device for treating wrinkles according to claim 1, **characterized in that** said programmable electronic unit (8) is configured to be controlled at little distance by a cellular telephone of smartphone kind (9) on which it is uploaded a suitable application configured to send the regulations parameters of said currents to said wireless transmission unit.

3. Portable device for treating wrinkles according to claim 1 or 2, **characterized in that** the respective position of said crown (4) and of said needled point (6) does not allow the needled point (6) to penetrate the skin beyond the area which can cause bleeding of the treated area.

4. Portable device for treating wrinkles according to any one of the preceding claims, **characterized in that a spring (5) is arranged inside the box at each electrode and is configured so that the respective electrode is pushed outwards** so that, when **the electrode (7)** is arranged on the skin in the area of the wrinkle to be treated, it is adapted to the surface irregularity thus always guaranteeing the contact between the electrodes (7) and the skin.

## Patentansprüche

1. Tragbare Vorrichtung zur Behandlung von Falten mittels amplitudenmodulierten, monodirektionalen Strömen, umfassend:
- ein äußeres Gehäuse (1) von solchen Abmessungen, dass es nur von einer Hand ergriffen werden kann,
- eine Mehrzahl von Elektroden (7) mit kleinen Abmessungen, die aus einer der Oberflächen des genannten Gehäuses (1) herauskommen, wobei jede der genannten Elektroden derart konfiguriert ist, dass sie entlang ihrer eigenen Achse innerhalb einer jeweiligen Führung (10) gleiten und auswärts durch die Wirkung einer Feder (5) geschoben werden kann;
- eine programmierbare elektronische Einheit (8), die innerhalb des genannten Gehäuses (1) angeordnet und derart konfiguriert ist, dass sie niederfrequente, amplitudenmodulierte, monodirektionale Ströme erzeugt und diese an die Elektroden (7) sendet;
- eine Batterie (2), die innerhalb des genannten Gehäuses (1) angeordnet und derart konfiguriert ist, dass sie die genannte programmierbare elektronische Einheit (8) speist,
**dadurch gekennzeichnet, dass**
jede der genannten Elektroden (7) in ihrem eigenen Endabschnitt mit einer kreisförmigen Krone (4) von spitzen Anschlüssen und mit einem anderen genadelten Punkt (6) versehen ist, der im wesentlichen auf der Achse der Elektrode (7) angeordnet und mit Bezug zu der Ebene der genannten spitzen kreisförmigen Krone (4) ragt,
und dass
die genannte Vorrichtung ferner eine drahtlose Datenübertragungseinheit (3) aufweist, die innerhalb der Box (1) angeordnet und derart konfiguriert ist, dass sie die Regulierungsparameter der genannten zu den Elektroden (7) gesandten Ströme verwaltet.

2. Tragbare Vorrichtung zum Behandeln von Falten nach Anspruch 1, **dadurch gekennzeichnet, dass** die genannte programmierbare elektronische Einheit (8) derart konfiguriert ist, dass sie in geringem Abstand von einem Mobiltelefon vom Smartphone-Typ (9) gesteuert wird, auf das eine geeignete Anwendung konfiguriert ist, um die Regulierungsparameter der genannten Ströme an die genannte drahtlose Übertragungseinheit zu senden.

3. Tragbare Vorrichtung zum Behandeln von Falten nach Anspruch 1 oder 2, **dadurch gekennzeichnet, dass** die jeweilige Position der genannten Krone (4) und des genannten genadelten Punkts (6) nicht zulässt, dass der genadelte Punkt (6) über den Bereich hinaus in die Haut eindringt, was zu Blutungen im behandelten Bereich führen kann.

4. Tragbare Vorrichtung zum Behandeln von Falten nach einem der vorhergehenden Ansprüche, **dadurch gekennzeichnet, dass** eine Feder (5) innerhalb der Box an jeder Elektrode angeordnet und derart konfiguriert ist, dass die jeweilige Elektrode nach außen derart ausgedrückt wird, dass wenn die Elektrode (7) auf der Haut im Bereich der zu behandelnden Falte angeordnet ist, wird sie der Oberflächenunregelmäßigkeit angepasst und garantiert somit immer den Kontakt zwischen den Elektroden (7) und der Haut.

## Revendications

1. Dispositif portatif de traitement des rides au moyen de courants monodirectionnels modulés en amplitude, comprenant:
- un boîtier extérieur (1) de dimensions telles qu'il peut être saisi seulement par une main,
- une pluralité d'électrodes (7) de petites dimensions qui sortent de l'une des surfaces dudit boîtier (1), chacune desdites électrodes étant configurée pour coulisser le long de son propre axe à l'intérieur d'un guide (10) respectif et être poussée vers l'extérieur par l'action d'un ressort (5);
- une unité électronique programmable (8), agencée à l'intérieur dudit boîtier (1), configurée pour générer des courants monodirectionnels à basse fréquence, modulés en amplitude, et pour les envoyer auxdites électrodes (7);
- une batterie (2), agencée à l'intérieur dudit boîtier (1), configurée pour alimenter ladite unité électronique programmable (8),
**caractérisé en ce que**
chacune desdites électrodes (7), dans sa propre partie terminale, est pourvue d'une couronne circulaire (4) de bornes pointues et d'un autre point aiguilleté (6) disposé sensiblement à l'axe de l'électrode (7) et faisant saillie au plan de ladite couronne circulaire pointue (4),
et **en ce que**
ledit dispositif comprend en outre une unité de transmission de données sans fil (3), agencée à l'intérieur du boîtier (1), configurée pour gérer les paramètres de régulation desdits courants envoyés aux électrodes (7).

2. Dispositif portable de traitement de rides selon la revendication 1, **caractérisé en ce que** ladite unité électronique programmable (8) est configurée pour être commandée à faible distance par un téléphone cellulaire de type smart-phone (9) sur lequel est chargée une application adaptée configurée pour envoyer les paramètres de régulation desdits courants à ladite unité de transmission sans fil.

3. Dispositif portatif de traitement des rides selon la revendication 1 ou 2, **caractérisé en ce que** la position respective de ladite couronne (4) et dudit point aiguilleté (6) ne permet pas au point aiguilleté (6) de pénétrer dans la peau au delà de la zone qui peut provoquer des saignements de la zone traitée.

4. Dispositif portatif pour le traitement des rides selon l'une quelconque des revendications précédentes, **caractérisé en ce qu'**un ressort (5) est disposé à l'intérieur du boîtier au niveau de chaque électrode et est configuré de telle sorte que l'électrode respective est poussée vers l'extérieur de sorte que, lorsque l'électrode (7) est disposée sur la peau dans la zone de la ride à traiter, elle est adaptée à l'irrégularité de surface garantissant ainsi toujours le contact entre les électrodes (7) et la peau.
